# EUROPEAN PATENT SPECIFICATION

(11) **EP 4 132 291 B1**
(45) Date of publication and mention of the grant of the patent: **07.10.2026**
(21) Application number: 21722336.1
(22) Date of filing: 07.04.2021
(51) Int. Cl.: A23K 50/40, G06Q 30/02, G06Q 50/10, G09B 19/00, G16H 20/60

(54) **METHODS FOR CHARACTERIZING THE NUTRITIONAL NEEDS OF AN ANIMAL AND FOR SELECTING FOOD PRODUCTS**
VERFAHREN ZUR CHARAKTERISIERUNG DER ERNÄHRUNGSBEDÜRFNISSE EINES TIERES UND ZUR AUSWAHL VON LEBENSMITTELPRODUKTEN
PROCÉDÉS PERMETTANT DE CARACTÉRISER LES BESOINS NUTRITIONNELS D'UN ANIMAL ET DE SÉLECTIONNER DES PRODUITS ALIMENTAIRES

(30) Priority: 07.04.2020 EP 20168471
(43) Date of publication of application: 15.02.2023
(73) Proprietor: Mars Incorporated, McLean, VA 22101-3885 (US)
(72) Inventor: ROCHE, Jean-Baptiste, 30470 Aimargues (FR); MONTOYA, Mathieu, 30470 Aimargues (FR); PEREA, Sally, Lewisburg, OH 45338 (US)
(74) Representative: Ipsilon
(86) International application number: PCT/US2021/026197
(87) International publication number: WO 2021/207370

(56) References cited:
- US-A1- 2014 141 134
- US-A1- 2015 072 048

## Description

### FIELD

The present invention relates to a computer-implemented method for characterizing the nutritional needs of an animal and customized and/or individualized animal food products, such as pet animal food products, selected through said computer-implemented method.

### BACKGROUND

There is an increasing awareness of the importance of a proper diet for the health maintenance and disease prevention or treatment of animals, especially of pets. Customized or individualized diets for various life stages and conditions are available for animals, especially for companion animals and pets. Although a high number of suppliers and products are available to pet care product consumers, it can take significant time, effort and investigation for a customer to determine which product(s) best suits a particular animal's need among the large number of alternatives. While veterinarians and other professionals can assist in recommending a given brand of pet food for a particular pet, pet foods are typically mass manufactured to meet the needs of an average pet within a given range, such as the age and/or size of the pet.

Nutritional needs, however, vary amongst pets, and a customized or individualized regimen of appropriate ingredients for a particular pet or breed of pet would be beneficial. In the light of the difficulties and expenses of tailoring a custom diet for a particular pet, consumers are often forced to choose between a limited variety of available mass-produced pet foods and products. Several manufacturers have conceived systems allowing the design of foodstuffs that better correlate with the specific nutritional requirements of their companion animal. For the purpose of example and not limitation, U.S. Patent No. 6,358,546 and U.S. Patent No. 6,493,641 are directed to methods for customizing or manufacturing pet food, in which a user input is used to form an individual pet profile used to generate a customized pet food formula.

For the purpose of example and not limitation, U.S. Patent Publication No. 2014/0272028 relates to systems and methods for collecting specific pet information and utilizing that information to create a custom pet food product. Based upon a correlation by a computer, a kibble or blend can be suggested from a number of possible pre-manufactured kibbles or blends, and a pet food additive can be created based on a specific pet food product formulation and in accordance with a pet profile of the pet.

International Publication No. WO 2014/078856 is directed to systems and methods for creating a customized blend of pet food. The methods, for example, include creating a first environment profile for the pet comprising the steps of inputting pet characteristics in a computer system, such as age, breed, weight and food preferences. The method also includes acquiring data, such as published pet nutrition information, comparing the data to the first environmental profile, generating the nutritional target from the data and the first environmental profile, and then generating a nutritional blend recipe using a computer system.

Despite the above, there remains a need for systems, methods, and/or apparatus for determining or providing a plurality of individualized food products that is adaptable and reproducible over time.

### SUMMARY

The invention pertains to the subject-matter of the claims.

The present invention concerns a computer-implemented method for characterizing the nutritional needs of an animal comprising:
providing an animal profile comprising one or more animal criteria;
extracting, from a first database, one or more lists of ingredients associated with the one or more animal criteria, wherein each one of the ingredients is assigned a range amount, wherein the range comprises a minimum amount and maximum amount, a minimum amount and a target amount, a maximum amount and a target amount, or a minimum amount, a maximum amount and a target amount;
determining a rating associated with the extracted lists of ingredients, wherein the rating provides the importance of the ingredients compared to the importance of other ingredients for the animal profile;
comparing the rating with at least one ingredient from the one or more lists of ingredients;
determining a nutritional profile of the animal based on the animal profile, the extracted lists of ingredients, and the rating of the extracted lists of ingredients.

In an embodiment of the invention the animal criteria are selected from: a species, an activity level, a medical history, a breed, a reproductive status, a feeding method, an age, a sex, a spayed or neutered status, a meal schedule, a biological value from a biological sample, a body condition, a health status, a life style, a coat information, a risk factor, and/or a weight of the animal.

In an embodiment of the invention the method comprises selecting food product(s) for an animal. The method includes providing a nutritional profile 160 of the animal. The method can also include providing a second database 170 including a plurality of pre-manufactured food products, each one being characterized by the individual content of the key ingredients comprising the pre-manufactured food products. In addition, the method can include selecting at least one pre-manufactured food products from the second database 180 based on the nutritional profile. In some embodiments of the invention the method can include ranking the selected pre-manufactured food product(s) 185, and/or providing a recommendation from the selected pre-manufactured food products.

Certain embodiments, not pertaining to the invention, are directed to a device for selecting food product(s) for an animal, having means adapted to or being configured to execute the steps of the methods for characterizing the nutritional needs of an animal, and/or for selecting food product(s) for an animal.

Certain embodiments, not pertaining to the invention, are directed to a computer program comprising instructions to cause the device to execute the steps of the method for characterizing the nutritional needs of an animal, and/or for selecting food product(s) for an animal.

Certain embodiments, not pertaining to the invention, are directed to a computer-readable medium having stored thereon the computer program.

A second aspect of the invention relates to a system comprising means for characterizing the nutritional needs of an animal according to the method of the invention, the system comprising:
a processor;
a storage unit;
a memory;
a user interface illustrated on a display; and
a first database, comprising one or more lists of ingredients associated with one or more animal criteria,
wherein the memory comprises instructions, which when executed by the processor, are configured to cause the system to:
   provide an animal profile comprising one or more animal criteria;
   extract, from the first database, the one or more lists of ingredients, wherein each one of the ingredients is assigned a range amount, defined by at least one of a minimum amount, a maximum amount, or a target amount;;
   determine a rating associated with the extracted lists of ingredients;
   comparing the rating with at least one ingredient from the one or more lists of ingredients;
   determine a nutritional profile of the animal, based on the animal profile and the one or more lists of ingredients.

### BRIEF DESCRIPTION OF THE DRAWINGS

**FIGURE 1A** illustrates a method or process for determining a nutritional profile of an animal.
**FIGURE 1B** illustrates a method or process for selecting at least one manufactured food product for an animal.
**FIGURE 2** illustrates a computer system.
**FIGURE 3A** illustrates a method or process for determining a nutritional profile of an animal.
**FIGURE 3B** illustrates a method or process for selecting at least one manufactured food product for an animal.

### DETAILED DESCRIPTION

As used herein, the words "a" or "an," when used in conjunction with the term "comprising" in the claims and/or the specification, can mean "one," but they are also consistent with the meaning of "one or more," "at least one," and/or "one or more than one". The terms "having," "including," "containing" and "comprising" are interchangeable, and one of skill in the art will recognize that these terms are open ended terms.

As used herein, the term "about" or "approximately" means within an acceptable error range for the particular value as determined by one of ordinary skill in the art, which will depend in part on how the value is measured or determined, *i.e.,* the limitations of the measurement system.

As used herein, the term "mammal" or "mammals" comprises a human or an animal. In particular, the term "animal" or "animals" can designate a ruminant, poultry, swine, mammal, horse, mouse, rat, rabbit, guinea pig, hamster, cow, cat or dog,.

As used herein, the term "adult" means an animal has passed puberty and reaches its biological maturation point.

As used herein, a "pet animal" can include a pet mammal. Pet mammals encompass dogs, cats, rabbits, hamsters, guinea pigs, rats and mice. Preferred pet animals herein can include feline or canine, especially as dogs and cats.

As used herein, the term "feline" encompasses animals, including pet animals, selected in the group comprising cheetah, puma, jaguar, leopard, lion, lynx, liger, tiger, panther, bobcat, ocelot, smilodon, caracal, serval and cats. As used herein, cats encompass wild cats and domestic cats.

As used herein, the term "canine" encompasses animals, including pet animals selected in the group comprising recognized dog breeds (some of which are further subdivided), which can include afghan hound, airedale, akita, Alaskan malamute, basset hound, beagle, Belgian shepherd, bloodhound, border collie, border terrier, borzoi, boxer, bulldog, bull terrier, cairn terrier, chihuahua, chow, cocker spaniel, collie, corgi, dachshund, dalmatian, doberman, English setter, fox terrier, German shepherd, golden retriever, great dane, greyhound, griffon bruxellois, Irish setter, Irish wolfhound, King Charles spaniel, Labrador retriever, lhasa apso, mastiff, newfoundland, old English sheepdog, papillion, pekingese, pointer, pomeranian, poodle, pug, rottweiler, St. Bernard, saluki, samoyed, schnauzer, Scottish terrier, Shetland sheepdog, shih tzu, Siberian husky, Skye terrier, springer spaniel, West Highland terrier, whippet, Yorkshire terrier, etc.

As used herein, a "subpopulation" is a set of one to multiple animals of one species, but less than an entire species, definable in terms of genotype and/or one or more attributes of physiological condition that, in a subpopulation of more than one member, are common to members of the subpopulation. In certain embodiments, the subpopulation is defined at least in part by specific breed. For example, in the case of animals of mixed breed, a subpopulation can be defined at least in part by breed heritage, which can be established through knowledge of the parental breeds, phenotypic characteristics, genotypic assessment, or by genetic markers such as single-nucleotide polymorphisms ("SNPs"). In certain embodiments, the subpopulation is defined at least in part by physiological condition.

As used herein, the term "animal profile" or "physiological profile" refers to any one or combination of attributes of an animal including its species, breed, reproductive status, sex, spayed or neutered status, size, weight, ideal body weight, Body Condition Score ("BCS"), age, activity level, lifestyle, coat information, disposition, state of wellness, feeding method, meal schedule, risk factor(s) and medical history. A physiological profile is a product of interaction of the genotype with the environment of the animal. A subpopulation defined at least in part by physiological profile can cut across breed lines. Alternatively, a subpopulation can be defined in part by physiological condition but restricted to one or a few breeds or a defined breed heritage. Examples of such subpopulations are aggressive poodles, Labrador retrievers with tapeworm infestation, spayed female dogs having a breed heritage that includes beagle, German Shepherds with hip dysplasia, Labrador Retrievers with obesity, Yorkshire terriers with chronic diarrhea (or protein loosing enteropathy), Chihuahua with dental disease, Rag doll with cardiac disease (or hypertrophic cardiomyopathy), Main Coon with osteoarthritis, Persian with calcium oxalate uroliths, etc.

From an algorithm point of view the risk factors and diseases are both criteria. However, a distinction can be made as risk factors relate to the notion of "prevention" whereas a disease relates to the management and reduction of clinical signs and progression of the disease. Risk factors are identified based on the information within the patient profile (such as, but not limited to, the medical history, breed, age, and body condition) that identify areas that should be considered within the animals nutrient/ingredient profile to help support maintenance of physiologic function (before the onset of disease). For example, the animal's breed (*e.g.*, large breed dogs) can predispose the animal to develop joint or mobility disease; therefore, the nutrient/ingredient profile includes consideration for the risk of joint/mobility conditions by controlled levels of dietary calcium and phosphorus, as well as moderate energy density (to promote slow and steady rate of growth) and including ingredients/nutrients such as, for example, glucosamine, chondroitin, and EPA+DHA.

In particular, the term "physiological profile" refers to any one or combination of attributes of an animal including its specie, breed, sex, neuter status, size, weight, ideal body weight, BCS, age, activity level, disposition, state of wellness, and medical history.

The term "pathological profile" is a particular type of animal/physiological profile, wherein the set of attributes further includes an indication for a set of pathological conditions which have been associated to a given animal, or which are suspected to be present in said animal. Thus, as used herein, the term "pathological profile" refers to any one or combination of diagnosed pathology(ies) or medical condition(s) as determined by a veterinary assessment including but not limited to clinical sign(s), veterinary exam(s), diagnostic blood work(s), diagnostic imaging (radiographs, ultrasounds), DNA test(s) and diagnostic cytology/biopsy.

For example, a subpopulation can include adult felines that shed hair excessively, obese dogs, toy dogs having respiratory disease, geriatric dogs of large breed type, long-haired cats having renal insufficiency, etc. Hence, the animal profile can include information pertaining to, but not limited to, an animal's name, species, age, weight, gender, breed, spayed/neutered, activity level, breeding status, digestive health, medical history and genetic information, current health status, body condition, feeding method, snack schedule and flavor preferences. Further, the animal profile can also include information regarding the season, date or time of year.

For the purpose of example, and no limitation, the disorders/pathologies listed in the public VENOM DATABASE (venomcoding.org) or in the public AAHA (American Animal Hospital Association) listing from the Veterinary Terminology Services Laboratory (https://vtsl.vetmed.vt.edu/aaha/) can be used, in part, for the establishment of a physiological or pathological profile. In certain embodiments, the following set of disorders/pathologies can be considered, in part, for the establishment of a physiological or pathological profile:

**Table 1 - Set of disorders/pathologies that can be considered for the establishment of a physiological or pathological profile.**

| | |
|---|---|
| Behavioral Disorders | Stress and Anxiety Related Disorders, Chronic Anxiety |
| Dermatological Disorders | Self-Induced Alopecia, Adverse Food Reactions (Afr), Adverse Food Reactions Suspicion, Atopic Dermatitis (Atopy), Dermatitis, Flea Bite Allergic Dermatitis, Pyoderma, Skin and Coat Disorders Wound Healing, Coat Color Disorder (Incl Red Coat Syndrome), Cutaneous Afr, Dermatosis, Hair Loss, Pyodermatitis |
| Endocrine Disorders | Diabetes Mellitus (Dm), Hypothyroidism, Diabetes Mellitus Type 1 (Absolute Insulin Deficiency), Diabetes Mellitus Type 2 (Relative Insulin Deficiency or Insulin Resistance), Hyperthyroidism, Thyroid Disorders |
| Food Intake Disorders | Anorexia, Dysrexia, Food Intake Disorders |
| Gastro-Intestinal Disorders | Acute Diarrhea, Acute Gastroenteritis, Acute Vomiting, Chronic Diarrhea , Chronic Enteropathy (Ce) , Chronic Gastroenteritis, Chronic Vomiting, Constipation, Fibre Responsive Colitis (Including Stress Diarrhea), Gastrointestinal Afr, Gastrointestinal Condition Requiring High Fibre Content, Inflammatory Bowel Disease (Ibd) , Intestinal Diseases, Lymphangiectasia , Maldigestion Malabsorption, Megacolon, Megaoesophagus, Perianal Fistula, Protein-Losing Enteropathy (Ple) , Acute Colitis, Antibiotic Responsive Diarrhea (Ard), Chronic Colitis, Chronic Idiopathic Large Bowel Disease (Cilbd) , Delayed Gastric Emptying, Feline Triaditis (Enteritis+Cholangiohepatitis+Pancreatitis), Food Responsive Diarrhea (Frd), Functional Colopathy , Gastric Dilatation Volvulus (Recurrence Prevention), Gastric Disorders, Gastritis, Obstipation, Oesophagial Disorders, Oesophagitis, Small Intestinal Bacterial Overgrowth (Sibo) , Steroid (Or Immunosuppressant) Responsive Diarrhea (Srd Or Ird) |
| Heart Disorders | Cardiovascular Diseases, Congestive Heart Failure (Chf), Hypertrophic Cardiomyopathy (Hcm), Dilated Cardiomyopathy (Dcm), Chronic Valvular Heart Disease (Cvhd) |
| Liver Disorders | Cholangiohepatitis, Cholangitis, Copper Storage Disease, Feline Hepatic Lipidosis, Hepatic Encephalopathy, Liver Diseases, Acute Hepatitis, Chronic Hepatitis, Liver Failure, Portosystemic Shunt (Pss) |
| Oral Disorders | Periodontal Disease, Dental Plaque, Gingivitis, Oral Diseases, Tartar |
| Osteoarticular Disorders | Bone Disorders, Joint Disorders, Osteoarthritis, OsteoArticular Disorders, Osteochondritis |
| Other | Regurgitation, Malnutrition, Food Intolerance (Including Gluten Intolerance), Hairball Management, Anemia, Hyperlipidemia (Fasting Hyperlipidemia), Neoplasia (Cancer), Neurologic Diseases, Convalescence, Hypertension, Sarcopenia |
| Pancreatic Disorders | Acute Pancreatitis, Chronic Pancreatitis, Exocrine Pancreatic Insufficiency (Epi), Pancreatic Disorders (Exocrine Pancreas), Chronic Pancreatitis (Mild Moderate), Chronic Pancreatitis (Severe) |
| Renal Disorders | Acute Kidney Injury (Aki), Azotemic Ckd Iris Stage 2, Azotemic Ckd Iris Stage 3, Azotemic Ckd Iris Stage 4, Ckd With Proteinuria, Early Ckd (Iris Stage 1), Chronic Kidney Disease (Ckd), Ckd Mineral Bone Disorder (Secondary Hyperparathyroidism), Ckd With Hypertension, Glomerulonephritis, Kidney Dysplasia, Polycystic Kidney Disease (Pkd), Protein-Losing Nephropathy (Pln) |
| Urinary Disorders | Feline Idiopathic Cystitis (Fic), Urinary Tract Infection (Uti), Urolithiasis Calcium Oxalate (Or Calcium Phosphate), Urolithiasis Cystine, Urolithiasis Struvite, Urolithiasis Urate (Or Xanthine), Hematuria, Incontinence, Proteinuria, Urinary Disorders, Urolithiasis Undetermined |
| Weight Disorders | Obesity, Overweight, Underweight, Cachexia , Obesity BCS 7 (Out Of 9), Obesity BCS 8 (Out Of 9), Obesity BCS 9 (Out Of 9), Overweight BCS 6 (Out Of 9), Underweight BCS 1 (Out Of 9), Underweight BCS 2 (Out Of 9), Underweight BCS 3 (Out Of 9) |

In certain embodiments, in dogs, such diseases and disorders can include, without limitation, adverse reactions to food (including food allergy and food intolerance), as can be manifested for example by one or more of the following: chronic colitis, chronic gastroenteritis, chronic otitis extrema or pruritic dermatitis; arthritis, including osteoarthritis; brain aging and related behavioral changes; cancer or neoplasia; cardiovascular disease, including ascites or edema (fluid retention), heart disease, heart failure, heartworm disease, and primary hypertension; developmental orthopedic disease; diabetes mellitus; gastrointestinal disorders, including colitis, fiber-responsive colitis, fiber-responsive constipation, constipation unresponsive to increased fiber, acute or chronic diarrhea, fiber responsive diarrhea, exocrine pancreatic insufficiency, flatulence, acute or chronic gastroenteritis, inflammatory bowel disease (IBD), maldigestion or malabsorption, non-hyper lipidemic pancreatitis, hyperlipidemic pancreatitis, recovery from gastrointestinal surgery, and acute or chronic vomiting, hepatic disorders, including ascites or edema (fluid retention), copper storage disease, hepatic encephalopathy, and liver disease, hyperlipidemia, obesity, oral health disorders, including gingivitis, oral malodor, and tartar, plaque or dental stain; recovery states, including anemia, anorexia, cachexia or weight loss, convalescence, debilitation, hyper metabolic states, malnutrition, and pre-surgical state and post-surgical state; renal disease, including hypertension, renal failure, and renal insufficiency; and urolithiasis, including calcium oxalate, urate and cystine management, struvite dissolution, struvite management, and struvite management in obese prone dogs.

In some embodiments, in cats, such diseases and disorders include, without limitation, adverse reactions to food (including food allergy and food intolerance), as can be manifested for example by one of the following: chronic colitis, eosinophilic granuloma complex, chronic gastroenteritis, or pruritic dermatitis; cardiovascular disease, including ascites or edema (fluid retention), heart disease, heart failure, and primary hypertension; diabetes mellitus; feline lower urinary tract disease, including idiopathic cystitis, oxalate management, struvite dissolution, struvite management, struvite management in obese cats, and Struvite management in obese prone cats; gastrointestinal disorders, including colitis, fiber-responsive colitis, fiber-responsive constipation, constipation unresponsive to increased fiber, acute or chronic diarrhea, fiber responsive diarrhea, acute or chronic gastroenteritis, IBD, pancreatitis, recovery from gastrointestinal surgery, and acute or chronic vomiting; hepatic disorders, including ascites or edema (fluid retention), copper storage disease, hepatic encephalopathy, and liver disease; hyperlipidemia; obesity; oral health disorders, including gingivitis, oral malodor, and tartar, plaque or dental stain; recovery states, including anemia, anorexia, cachexia or weight loss, convalescence, debilitation, hypermetabolic states, malnutrition, and pre-surgical state and post-surgical state; renal disease, including hypertension, renal failure, and renal insufficiency; and urolithiasis, including calcium oxalate, urate and cystine management, struvite dissolution, struvite management, and struvite management in obese prone cats.

As used herein the "ideal body weight" refers to the measured mass of the animal or the ideal mass of the animal as defined in a unit of weight such as kilograms or pounds. The BCS can be a measure of the animal based on the shape of the animal's body from a visual assessment from the side and from above the animal when in a standing position. The BCS assessment can also include a tactile assessment using one's hands to feel the level of the fat mass over the animal's ribs (assessment based on how easily or difficult to palpate the ribs). The BCS can refer to a score on a scale which typically ranges from 1 to 5, or 1 to 9, with 1 representing the lowest body weight and 9 representing the highest body weight. In certain embodiments, the BCS relates to the 1 to 9 scale, with a score of 5 being an ideal body weight.

As used herein, the term "biological sample" can refer to, but is not limited to, at least one of stool, urine, hair, blood, saliva, and tissue. Indeed, the term, "biological sample" refers not only to the biological material itself (proteins, nucleic acids, tissues, etc.) but also to other materials associated therewith used for detection of the biological material, or portions thereof (*e.g.,* dyes, labels, stains, or any other marker used in the identification of materials).

For example, a biological material can refer to a sample of tissue or fluid isolated from a subject, including but not limited to, for example, blood, plasma, serum, fecal matter, urine, bone marrow, bile, spinal fluid, lymph tissue and lymph fluid, samples of the skin, external secretions of the skin, respiratory, intestinal, and genitourinary tracts, tears, saliva, milk, blood cells, organs, biopsies and also samples of in vitro cell culture constituents including but not limited to conditioned media resulting from the growth of cells and tissues in culture medium, *e.g*., recombinant cells, and cell components. A biological sample can include, for instance, a polypeptide or a polynucleotide, or fragmented portions of organisms or cells obtained from sampling the environment, such as airborne pathogens.

As used herein, the term "preventing", can also include the reduction of a likelihood of occurrence, or of re-occurrence, of a given condition in an animal.

As used herein, the term "food product" or "food composition" or "diet" or "foodstuff" can cover all of foodstuff, diet, food supplement, liquid or a material that can contain proteins, carbohydrates and/or crude fats. Foods can also contain supplementary substances or additives, for example, minerals, vitamins and condiments (*See* Merriam- Webster's Collegiate Dictionary, 10th Edition, 1993). Such food compositions or products can be nutritionally complete or not.

In some embodiments, a therapeutic diet or a dietetic diet could be a food composition for the good health of urinary system, *e.g*. Renal Select Feline or Instinctive, produced by Royal Canin^{®}.

As used herein, the term "nutritionally complete" can designate a composition *(i.e.,* diet or foodstuff) that provides the complete and balanced nutritional requirements to the target animal *(i.e.,* the pet animal). For instance, such a nutritionally complete composition can be a complete dog or a complete cat food. A nutritionally complete dry composition is such that it can nutritionally satisfy the pet animal, *e.g.* a dog, can be fed as a daily ration *(i.e.,* which is capable of sustaining life without additional food, except water). In some embodiments, a nutritionally complete food composition can include, in a non-exclusive manner, one or more of cereals and vegetable protein extracts, fibers, oils and fats, proteins, chicory pulps, yeasts and parts thereof, minerals, vitamins, preservatives, antioxidants, water, amino-acids, sodium. A nutritionally complete compositions, for example, can include at least one source of proteins (or polypeptides or amino-acids), such as protein extracts, at least one source of vitamins, at least one source of fats (or fatty acids) and at least one source of minerals.

As used herein, a "pet food" or "animal food" refers to any product produced by a pet food manufacturer, whether processed, partially processed or unprocessed, intended to be ingested by pet animals after placing on the market. "pet food" or "animal food" in certain embodiments can be defined according to European Union (EU) Regulation n°767/2009.

As used herein, a "complete pet food" can be defined according to the European Union (EU) Regulation n°767/2009, on the placing on the market and use of feed (art. 3(i)) adapted to pet food). If a manufacturer labels a product as a complete pet food without specification of a determined life stage, it is assumed to be complete for all life stages, and should be formulated according to the levels recommended *(i.e.,* for early growth and reproduction). If the product is designed for a specific life stage, then the label must clearly state this.

As used herein, the term "hypoallergenic diet" or "anallergic food" refers to a food composition compatible with an allergen-restricted diet. These hypoallergenic diets can be unpalatable to or inappropriate for certain animals, causing the animal to develop an aversion to this food composition.

As used herein, a "meal schedule" refers to the frequency of meals that can be served to a target animal, which would remain consistent with the expected daily ration to be served to the animal.

As used herein, a "daily ration" represents the average total quantity of feeding stuffs, required daily by an animal of a given species, age category and yield, to satisfy its needs. The average total quantity of a specific pet food that is needed daily by a pet of a given species, age category, life style, and/or activity can be used to satisfy the energy and ingredient requirements of the animal. In certain embodiments "daily ration" or "needed daily" can be as defined in EU regulation n°767/2009 and use of feed (art. 2(c)).

As used herein, a "complementary pet food', also by reference to EU regulation n°767/2009 and use of feed (art. 3(j)) adapted to pet food, is a pet food which has a high content of certain substances but which, by reason of its composition, is sufficient for a daily ration only if used in combination with other pet foods.

As used herein, an "Allowance" or "Recommendation for daily intake (RDI)" is the level of intake of an ingredient or food component that appears to meet the known nutritional needs of practically all healthy individuals. It reflects the minimum requirement plus a safety margin for differences in availability between individual animals and for ingredient interactions. In practice this would be translated as the levels of essential nutrients that healthy individuals should consume over time to ensure adequate and safe nutrition.

As used herein, a "nutrient requirement" or "ingredient requirement" is the quantity of a nutrient/ingredient that must be supplied to an animal in order to satisfy its metabolic needs. It reflects the minimum average level of intake of an ingredient, which, over time, is sufficient to provide the needed nutrients to maintain the desired biochemical physiological functions in a population.

As used herein, an amount of a component as expressed as weight/Mcal comprising a weight amount of the component by unit of Metabolizable Energy ("ME") of the total food composition.

As used herein, the "Metabolizable Energy" refers to the digestible energy minus the energy lost in urine and combustible gases.

As used herein the "Maintenance Energy Requirement ("MER")" is the energy required to support energy equilibrium, where the ME can equal heat production over a long period of time.

In certain embodiments, the ME value can be measured using feeding trial. In practice, the gross energy (GE) of the food is determined in the laboratory, and the amounts of food eaten by the animals are recorded. The feces and urine from the animals are collected, and the energy in each is determined and called faecal energy (FE) and urinary energy (UE), respectively. The ME is then calculated as: ME (kcal/kg) = [GE - (FE + UE)] / (Kg of food consumed). Metabolizable Energy is determinable according to standard methods, and especially according to the European Standard EN 16967 (ICS.65.120) dated July 2017. Hence, as used herein, the term *"x g*/*Mcal"* for a given substance comprised in a diet or foodstuff means that the substance is comprised in an amount of x grams per Mcal contained in the diet of foodstuff.

Assuming an energy density of 16.7 kJ (4 kcal) ME per gram of dry matter, the following conversion factors can be applied: $Units / 100 g of dry matter \times 2.5 = units / 1000 kcal .$

As used herein, the term "ppm" or "ppm units" (also termed *"parts per million*"), is a way of specifying an amount of a substance comprised in a composition/product. It can be used herein as an amount of a substance per mass of the composition/product unless stated otherwise. Hence, the term ppm can refer to an amount (mg) per mass (kg) of the composition/product.

As used herein, a "pre-made composition" refers to a composition which is suitable for food consumption by an animal, which can be nutritionally complete or not.

As used herein, the term "wet food" as in "wet food composition" can refer to a food composition having a moisture content of 30% or more, a food composition of more than 40% by weight, relative to the total weight of the food composition, or the wet food composition has a moisture content lower than 90% in weight, relative to the total weight of the food composition. It can be the final product of a process comprising a final step of sterilization (instead of a drying step). In certain embodiments, the wet food comprises a chunk form, more particularly of chunks in gravy form. The wet food can comprise chunks and gravy, chunks in jelly, loaf, mousse, terrine, and/or bites form. "Chunks and gravy" products can comprise a preformed meat particle prepared by making a meat emulsion and by putting this meat emulsion through a muzzle under pressure and then cooked. A product, such as cooked meat, can be diced into chunks, which can be mixed with a gravy or sauce. The two components can then be filled into a container, which can be a can or pouch, which can be seamed or sealed and sterilized. As opposed to the ground loaf, chunk and gravy compositions can have physically separated, discrete chunks *(i.e.,* pieces of ground meat and grains) as prepared. These discrete particles can be present in the gravy-type liquid in the final container. When serving, chunk and gravy products can flow out of the can and can be easily mixed with other dry products. While the chunk and gravy products allow better integrity of the individual ingredients, the heterogeneous formulation of the chunk and gravy products are sometimes disfavored by consumers. Wet food compositions can be packaged in can-like containers and are considered "wet" in appearance because of the moisture contained therein. Certain embodiments can include a type of wet composition known as a "ground loaf." Loaf products are typically prepared by contacting a mixture of components under heat to produce a mostly homogeneous, intracellular honeycomb-type mass also known as a "ground loaf." The ground loaf mass is then packaged into a cylindrical container, such as a can. Upon packing, ground loaf can assume the shape of the container such that the ground loaf must be cut when serving to a companion animal. The wet food composition can be packaged. **In** this way, the consumer is able to identify, from the packaging, the ingredients in the food product and confirm that it is suitable for the particular pet in question. The packaging can be metal, plastic, paper or card.

As used herein, the term "dry food" as in "dry food composition" can refer to a food or composition having a moisture content of less than 12% by weight, relative to the total weight of the food composition, and commonly even less than 7% by weight, relative to the total weight of the food composition. In certain embodiments, dry food according to the present disclosure has a moisture content of at most 12% by weight. In some embodiments, the dry food has a moisture content of 7% or less, such as 5% by weight. In certain embodiments, the dry food has a moisture content of more than 3% by weight, relative to the total weight of the food composition. For instance, the examples provided herein illustrate a dry food having a moisture content of 9.5% by weight, relative to the total weight of the food composition. In certain embodiments the dry food can include a kibble. For example, and without limitation, kibbles include particulates; pellets; pieces of pet food, dehydrated meat, meat analog, vegetables, and combinations thereof; and pet snacks, such as meat or vegetable jerky, rawhide, and biscuits. The dry food composition can be manufactured by mixing together ingredients and kneading in order to make consistent dough that can be cooked. In general, it can be the final product of a process comprising an extrusion step followed by a drying step. According to some embodiments, it can be the final product of a process comprising an extrusion step followed by a coating step.

The process of creating a dry food can be done by baking and/or extruding. The dough is typically fed into a machine called an expander and/or extruder, which uses pressurized steam or hot water to cook the ingredients. While inside the extruder, the dough is under extreme pressure and high temperatures. The dough can be pushed through a die (specifically sized and shaped hole) and then cut off using a knife. The puffed dough pieces are made into kibble by passing it through a dryer so that moisture is dropped down to a defined target ensuring stability of the food until consumption. The kibble can then be sprayed with fats, oils, minerals, vitamins, the natural extracts cocktail and optionally sealed into packages. The dry food composition can be packaged. In this way, the consumer is able to identify, from the packaging, the ingredients in the food product and confirm that it is suitable for the pet, *e.g.* dog, in question. The packaging can be metal, plastic, paper or card.

As used herein, the term "semi-moist food" or "semi-moist food composition" can refer to a food composition with an intermediate moisture content of about 12% to about 30% in weight, relative to the total weight of the food composition. Hence, such semi-moist food composition can be the final product of a process allowing a moisture content value that is intermediate between a dry food and a wet food. In some embodiments, the process can comprise a step of adding a humectant agent. In some embodiments, the process comprises an extrusion step and a subsequent treatment step with Super-Heated Steam ("SHS"). In some embodiments, the semi-moist food according to the present disclosure containing more than 12% and at most 30% moisture by weight, relative to the total weight of the food composition. In certain embodiments, a semi-moist food composition has 11% to 25% moisture by weight, relative to the total weight of the food composition, and/or a water activity of 0.64 to 0.75.

As used herein, a *"nutritional maximum limit"* is the maximum level of an ingredient in a complete pet food that, based on scientific data, has not been associated with adverse effects in healthy pets, in particular healthy dogs and cats. The adverse effects in healthy pets can be based on the European Pet Food Industry Federation Guidelines. In addition, maximum permitted levels have been determined by the legislator for several ingredients if added as a nutritional additive (*i.e.,* trace elements & vitamin D) (legal maximum). They are laid down in the Community Register of Feed Additives pursuant to Regulation 1831/2002/EC of the Parliament and the Council, concerning additives in feeding stuffs. The legal maximum levels apply to all life stages (EU Regulation 1831/2003 in conjunction with EU register of feed additives).

### Methods for characterizing nutritional needs & for selecting food product(s)

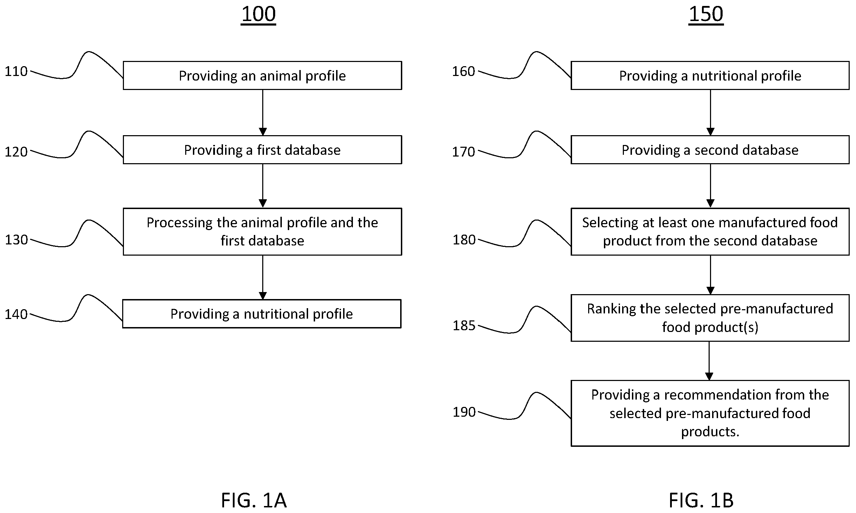

**FIGURE 1A** illustrates a first embodiment of the invention, relating to a computer-implemented method for characterizing the nutritional needs of an animal. The method comprises providing an animal profile 101, including a plurality of criteria, for instance selected from: a species, an activity level, a medical history, a breed, a reproductive status, a feeding method, an age, a sex, a spayed or neutered status, a meal schedule, a biological value from a biological sample, a body condition, a health status, a life style, a coat information, a risk factor, a weight of the animal; providing a first database 120, comprising a list of key ingredients to each animal criteria, each one of the key ingredients being assigned a range of amount, defined by a minimum amount and maximum amount, a minimum amount and a target amount, a maximum amount and a target amount, or a minimum amount, a maximum amount and a target amount, and for each range, a rating providing the importance of the ingredients compared to the importance of the other key ingredients listed in the first database; processing the animal profile 130 and the first database, thereby providing a nutritional profile 140 of the animal.

Some or all of the plurality of criteria can be associated with a weight factor. Such a "weight factor", or "weighting factor", is a weight given to a data point (in this case a set of nutritional recommendations associated to a given animal criteria or group of animal criteria) to assign it a lighter, or heavier, importance relatively to another data point, or group of data points *(i.e.,* a set of nutritional recommendations associated to another given an animal criteria or group of animal criteria).

The weight factor can be used for calculating a weighted mean, to give less (or more) importance to group members. Hence, certain embodiments relate, in particular, to a method for characterizing the nutritional needs of an animal, which can include the steps of: providing an animal profile, including a plurality of criteria, some or all being associated with a weight factor; extracting from, a first database 120, a list of key ingredients to each animal criteria, each one of the key ingredients being assigned a range of amount, defined by a minimum amount and/or a maximum amount and/or a target amount, and for each range, a rating providing the importance of the ingredients compared to the importance of the other key ingredients listed in the first database 120; processing the animal profile and the first database, thereby providing a nutritional profile of the animal. According to an one embodiment, one or more of the criteria, from the plurality can thus be prioritized over one or more other animal criteria considered for the animal profile. Hence, according to one such embodiment, the prioritized one or more animal criteria can overrule the non-prioritized animal criteria.

**FIGURE 1B** illustrates a second exemplary embodiment of the invention, relating to a method for selecting food product(s) for an animal. In certain embodiments of the invention, the method includes: providing a nutritional profile 160 of the animal according the above-mentioned method of characterizing the nutritional needs of the animal; providing a second database 170, comprising a plurality of pre-manufactured food products, each one being characterized by the individual content of the key ingredients composing the pre-manufactured food products; selecting at least one pre-manufactured food products from the second database 180 based on the nutritional profile; ranking the selected pre-manufactured food product(s) 185; and providing a recommendation 190 based on the selected pre-manufactured food products. The recommendation 190 can further be displayed on a user display or graphical user interface, in part to assist a user with purchasing the recommended and/or to determine potential food products for purchase. The food products can be any known animal food or pet food.

Advantageously, the first and second embodiments can be combined. Accordingly, certain embodiments of the invention also relate to a method for selecting food product(s) for an animal. In certain embodiments the method can include: providing an animal profile 110, including a plurality of criteria, selected from: a species, an activity level, a medical history, a breed, a reproductive status, a feeding method, an age, a sex, a spayed or neutered status, a meal schedule, a biological value from a biological sample, a body condition, a health status, a life style, a coat information, a risk factor, a weight of the animal; extracting, from a first database 120, a list of key ingredients to each animal criteria, each one of the key ingredients being assigned to a range defined by a minimal amount and a maximal amount, and for each range, a rating providing the importance of the ingredients compared to the importance of the other key ingredients listed in the first database; processing the animal profile and the first database 130, thereby providing a nutritional profile 140 of the animal; extracting, from a second database 170, a plurality of pre-manufactured food products, each one being characterized by the individual content of the key ingredients composing the pre-manufactured food products; selecting at least one pre-manufactured food products from the second database 180 based on the nutritional profile; ranking the selected pre-manufactured food product(s) 185; providing a recommendation on the selected pre-manufactured food products 190.

In certain embodiments of the invention, the method can select a plurality of pre-manufactured food products from the second database based on the nutritional profile. In certain embodiments of the invention, the pre-manufactured food products are commercial products. In one exemplary embodiment, the methods described herein are nutritionally complete. In certain embodiments of the invention, the pre-manufactured food products are selected from a group comprising: dry food products, wet food products, semi-moist food products, treats, Functional Meal Enhancers ("FME"). In certain embodiments, the animal is a pet. In certain embodiments of the invention, the animal is a dog or a cat.

In certain embodiments of the invention, the one or more values indicative of a body condition are selected from the group comprising: animal's breed, animal's age, animal's actual weight, animal's targeted weight, animal's BCS, animal's activity, animal's lifestyle, animal's sexual status, animal's gestation status.

In certain embodiments, the animal is a cat and the one or more values indicative of a health status can be selected from the following group: Post weight loss, Overweight, Obesity, Osteoarthritis, Mobility risk factors, Chronic Kidney Disease (CKD) Stage I or II, CKD Stage III or IV, Proteinuria, Struvite urolith dissolution, Struvite urolith prevention, CaOx prevention, CaP prevention, Idiopathic cystitis, Poor skin and coat, Atopy, non-food related dermatopathies, Dental Calculus, Acute or chronic diarrhea, Acute or chronic vomiting, Gastritis, Enteritis, Colitis, Maldigestion, Malabsorption, Diabetes mellitus, Pancreatitis, Exocrine pancreatic insufficiency ("EPI"), and/or Hyperlipidemia.

In certain embodiments, the animal is a dog and the one or more values indicative of a health status are selected from the group comprising: Post weight loss, Overweight, Obesity, Osteoarthritis, Mobility risk factors, CDK Stage I or II, CKD Stage III or IV, Proteinuria, Struvite urolith dissolution, Struvite urolith prevention, CaOx prevention, CaP prevention, Idiopathic cystitis, Poor skin and coat, Atopy, non-food related dermatopathies, Dental Calculus, Acute or chronic diarrhea, Acute or chronic vomiting, Gastritis, Enteritis, Colitis, Maldigestion, Malabsorption, Diabetes mellitus, Pancreatitis, Exocrine pancreatic insufficiency (EPI), Hyperlipidemia, Adverse food reaction.

A first database 220 can refer to a database comprising a list of key ingredients to each animal criteria. The key ingredients can be assigned to a range of amount, defined by a minimum amount and maximum amount, a minimum amount and a target amount, a maximum amount and a target amount, or a minimum amount, a maximum amount and a target amount. For each range, a rating providing the importance of the ingredients can be compared to the importance of the other key ingredients listed in the first database 220.

Non-exhaustive lists of key ingredients according to certain embodiments of the invention are provided in tables 3 to 5 and 7 to 9. Non-exhaustive lists of ranges defined by a minimal amount and a maximal amount corresponding to each one of the listed key ingredients can also be provided in tables 3 to 5 and 7 to 9. Such ranges can be expressed in the form of a Maintenance Energy Requirement (MER).

A "range", as defined herein thus comprises at least a minimal amount and a maximal amount; according to one particular embodiment, each range is further associated to a "target" amount, as further provided in tables 3 to 5 and 7 to 9.

A "target" amount can comprise of an amount associated to scientific evidence. A target can also be a target value, or a maximum or a minimum value to be found in a selection of food products or food compositions, or even of a plurality of food products or compositions from the second database, such as a portfolio of commercial food product (*i.e.,* a RoyalCanin^{©} Portfolio). When there is no precise evidence or when the evidence is only based on a threshold, an "optimum target" can be generated.

For instance, such a "target" amount can be defined as an amount comprised between a minimal amount and a maximal amount associated to a particular nutritional profile. According to some embodiments, the target amount equals the minimal or the maximal amount.

A minimum amount, a maximum amount and/or a target amount can be thus defined in the first database 220 by reference to a discrete value, such as a mean or median value from a plurality of values, which can be a plurality of values from a plurality of pre-manufactured food products belonging to the second database 230. Thus, according to one embodiment, the range of amount is defined by a minimal amount and a maximal amount and a target amount. According to one embodiment, the range of amount is defined by a minimal amount and a target amount. According to one embodiment, the range of amount is defined by a maximal amount and a target amount. An embodiment for the target amount calculation is described hereafter.

In Table 1 below x can represent specific value defined, null can represent no defined value, maxF can represent a maximum value found in a database of pre-manufactured food products, such as RoyalCanin^{®} Portfolio (specific for a species, cat or dog), and MinF can represent minimum value found in the database of pre-manufactured food products, such as RoyalCanin^{®} Portfolio.

| **Min** | **Max** | **Target** | | **Optimum Target** |
|---|---|---|---|---|
| x | x | null | | = (max+min) /2 |
| x | null | null | | = maxF |
| null | x | null | | = max / 2 |
| x | null | x | | = target |
| null | x | x | | = target |
| x | x | x | | = target |
| x | null | min | | = Min (x) |
| null | x | min | | = minF |
| x | x | min | | = Min (x) |
| x | null | max | | = MaxF |
| null | x | max | | =Max (x) |
| x | x | max | | =Max (x) |

### Table 1 - Target Calculation

For each key ingredient, a maximum amount (max), a minimum amount (min), a target amount, and a rating, providing the importance of the ingredients compared to the importance of the other key ingredients, are attributed. Two fields, for example, can include a first field selected from at least one of a minimum amount, a maximum amount, and/or a target amount, and a second field being a rating. The two fields, for example, include at least one target amount and one rating for each key ingredient.

The rating providing the importance of one or more ingredients can be compared to the rating of the one or more other key ingredients listed in the first database 220, based on the nutritional profile. In certain embodiments, the rating can be further modulated by the content of the individual physiological profile and/or the content of the individual pathological profile and/or the animal profile.

According to certain embodiments, the physiological or pathological or animal profiles can be determined based on characteristics which are, in part or totally, associated to contradictory recommendations, in which case the assigned rating will be modulated by an algorithm accordingly. Particular ways of modulating the ratings can thus be achieved by generating an optimum profile, based on the animal profile or the nutritional profile.

According to certain embodiments, an optimum profile is generated by modulating the minimal amount, the maximal amount, the target amount and/or the rating of one or more key ingredients from the first database 220. According to certain embodiments, one or more animal criteria might be prioritized against others. Alternatively, all criteria can be considered for generating the optimum profile.

Advantageously, the target values and weight factors from the optimum profile can also be used to rank the pre-manufactured food products from the second database 230. For instance, the min, max and weight from optimum profile can be used in the calculation of overall percentage of coverage and the criteria's percentage of coverage of the products.

In certain embodiments a second database 230 comprising a plurality of pre-manufactured food products, each one being characterized by the individual content of the key ingredients composing the pre-manufactured food products. Some examples of food products, such as animal food or pet food, which can be stored in second database 230 can be found in https://www.royalcanin.com/fr/cats/products/retail-products, https://www.royalcanin.com/fr/cats/products/vet-products, https://www.royalcanin.com/fr/dogs/products/retail-products, https://www.royalcanin.com/fr/dogs/products/vet-products, https://www.petsmart.com/dog/food/dryfood/#page_name=flyout&category=dog&cta=dryfood, https://www.zooplus.fr/shop/chiens/croquettes_chien, and/or https://www.chewy.com/.

Advantageously, certain embodiments can select a plurality (*i.e.,* two or more) food products from the second database 230. In some embodiments, the selected pre-manufactured food product(s) can be ranked. In one embodiment of the invention, the methods described herein include a step of ranking the selected pre-manufactured food products. The step of ranking the selected pre-manufactured food product can be achieved by: assigning to one or more of the selected pre-manufactured food products a quality factor based on the nutritional profile, and then ranking the selected pre-manufactured food product(s) of the second database based on the quality factor, by reference to non-selected food products of the second database; and/or assigning to one or more of the selected pre-manufactured food products a quality factor based on the nutritional profile, and then ranking the selected pre-manufactured food product(s) of the second database based on the quality factor, by reference to non-selected food product(s) of a distinct database.

In one embodiment of the invention, the methods disclosed herein include a step of providing a food recommendation on the selected pre-manufactured food products. In one embodiment of the invention, the step of providing a recommendation on the selected pre-manufactured food products can be achieved by assigning to one or more of the selected pre-manufactured food products a recommendation based on the nutritional profile. In a non-exhaustive manner, this recommendation can comprise a recommendation on the level of intake of an ingredient or food component that appears to satisfy or meet the known nutritional needs of the animal.

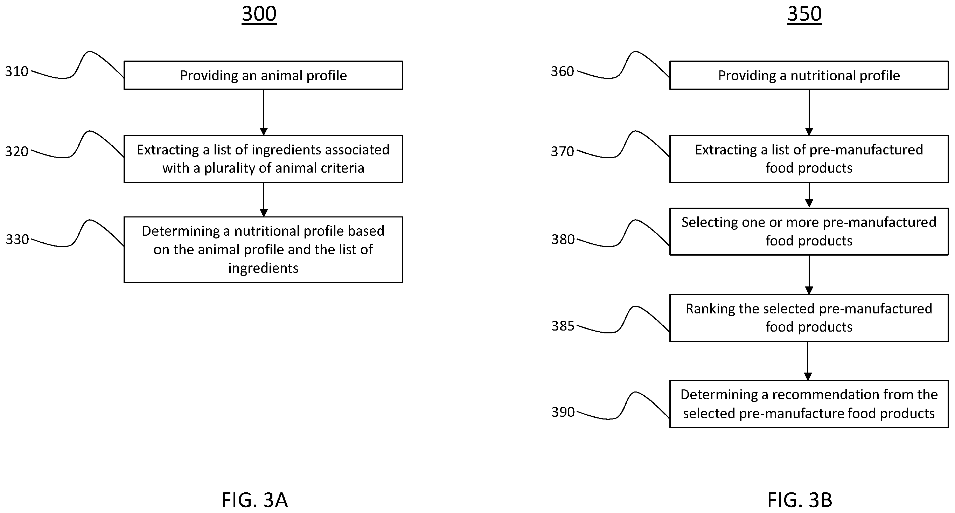

**FIGURE 3A** illustrates a method or process 300 for determining a nutritional profile of an animal according to certain embodiments of the invention. In certain embodiments, the method can include providing an animal profile including one or more criteria, as shown in 310. The one or more criteria, for example, can include a species, an activity level, a medical history, a breed, a reproductive status, a feeding method, an age, a sex, a spayed or neutered status, a meal schedule, a biological value from a biological sample, a body condition, a health status, a life style, a coat information, a risk factor, or a weight of the animal. As shown in 320, a list of ingredients can be extracted from a first database. The list of ingredients can be associated with each animal criteria. Each of the ingredients can be assigned a range of amounts. The range of amounts, for example, can include at least one of a minimum amount, a maximum amount, and/or a target amount. In certain embodiments, a rating can be provided for the one or more ingredients. The rating, for example, can be associated with the importance of a given ingredient compared to the importance of one or more other ingredient listed in the first database. In some embodiments of the invention, a nutritional profile can be determined based on the animal profile and the list of ingredients, as shown in 330. This determination can be based on a variety of factors and performed in numerous ways. In certain embodiments of the invention, a plurality of nutritional criteria are generated, with each criteria assigned a criteria value. Each of these nutritional criteria can then be compared to the list of ingredients associated with a plurality of animal criteria. Each criteria can further or alternatively be assigned a prioritization value which indicates which criteria should be given priority over others. Finally, a nutrition profile can be calculated, or displayed, based on a combination of the list of ingredients, the criteria values, and the prioritization values. The nutritional profile can further be output on a user display or otherwise exported.

**FIGURE 3B** illustrates a method or process 350 for selecting at least one manufactured food product for an animal according to certain embodiments of the invention. In certain embodiments of the invention, the method or process can include providing a nutritional profile of the animal base the characterized nutritional needs of the animal, as shown in step 360. In step 370, the method can include extracting from a second database 370, one or more food products, such as pre-manufactured food products. Each of the food products can be characterized by the ingredients included within the pre-manufactured food products. At least one pre-manufactured food product can be selected from the second database based on the nutritional profile, as shown in 380. The selected pre-manufactured food product(s) can be ranked, in step 385, and/or a recommendation can be provided in step 390 based on the selected pre-manufactured food products. The recommendation, for example, can further be displayed on a user display or graphical user interface. In certain embodiments of the invention, the user can purchase the recommended food product.

### Devices, Computer Systems, Apparatus, and Computer-Implemented Methods & Mediums

The invention is restricted to the subject-matter of the claims. Some embodiments, not pertaining to the invention, relate to a device for characterizing the nutritional needs of an animal and/or for selecting food product(s) for the animal, being configured to or having means adapted to execute the steps of the methods of certain embodiments.

Other embodiments, not pertaining to the invention, relate to a computer program comprising instructions to cause the device according to the preceding claim to execute the steps of the methods of certain embodiments.

Other embodiments, not pertaining to the invention, relate to a computer-readable storage medium having stored thereon the computer program described above. Such a computer-readable medium can comprise a physical embodiment of a collection of data comprising one to a plurality of data sets that can be configured in one to a plurality of databases. It can further comprise a medium wherein or whereon such data is/are stored. Such a computer-readable medium can also comprise more than one medium; however in such a case the media are functionally linked.

A computer-aided system according to the invention comprises one or more user interface(s) 210 enabling entry of an input data. Such input data can, for instance, comprise means for providing one or more databases by the user, such as for instance all or parts of the first and second databases which are parts of the methods according certain embodiments of the invention. The system further comprises a processor, a storage unit, and a memory, wherein the memory comprises instructions, which when executed by the processor, are configured to cause the system to:
provide an animal profile comprising one or more animal criteria;
extract, from the first database, the one or more lists of ingredients, wherein each one of the ingredients is assigned a range amount, defined by at least one of a minimum amount, a maximum amount, or a target amount;
determine a rating associated with the extracted lists of ingredients;
comparing the rating with at least one ingredient from the one or more lists of ingredients;
determine a nutritional profile of the animal, based on the animal profile and the one or more lists of ingredients.

Advantageously, such a system can be further configured to provide and/or manufacture the selected pre-manufactured food products from the second database. Further, the device can be configured to purchase the selected pre-manufactured food products from an external website.

In certain embodiments, such manufacture can be in part or in whole under the control of, and/or driven by, an extension of the computer-aided system from a range of pre-existing options, *e.g.,* an existing pet food product line, to best fit or match the nutritional formula.

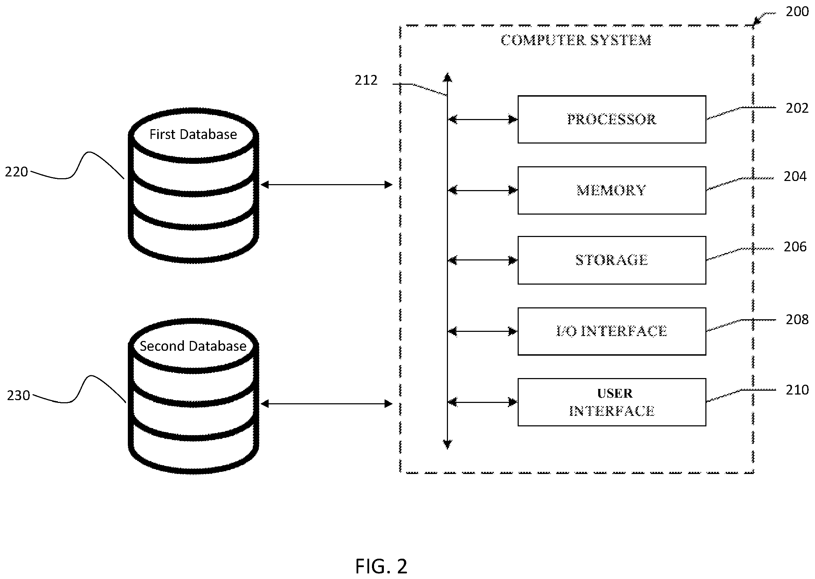

Some embodiments can be implemented in digital electronic circuitry, or in computer software, firmware, or hardware, including the structures disclosed in this specification, such as shown in FIG. 2, and their structural equivalents, or in combinations of one or more of them. Certain embodiments can be implemented as one or more computer program products, *i.e.,* one or more modules of computer program instructions encoded on a computer-readable medium for execution by, or to control the operation of, data processing apparatus. The computer-readable medium can be a machine-readable storage device, a machine-readable storage substrate, a memory device, a composition of matter effecting a machine-readable propagated signal, or a combination of one or more of them. The term "data processing apparatus" encompasses all apparatus, systems, such as computer system 200, devices, and machines for processing data, including by way of example a programmable processor, a computer, or multiple processors or computers. The apparatus can include, in addition to hardware, code that creates an execution environment for the computer program in question, *e.g*., code that constitutes processor firmware, a protocol stack, a database management system, an operating system, or a combination of one or more of them. A propagated signal is an artificially generated signal, *e.g.,* a machine-generated electrical, optical, or electromagnetic signal, that is generated to encode information for transmission to suitable receiver apparatus.

A computer program (also known as a program, software, software application, script, or code) can be written in any form of programming language, including compiled or interpreted languages, and it can be deployed in any form, including as a stand-alone program or as a module, component, subroutine, or other unit suitable for use in a computing environment. A computer program does not necessarily correspond to a file in a file system. A program can be stored in a portion of a file that holds other programs or data (*e.g.,* one or more scripts stored in a markup language document), in a single file dedicated to the program in question, or in multiple coordinated files (*e.g.,* files that store one or more modules, sub-programs, or portions of code). A computer program can be deployed to be executed on one computer or on multiple computers that are located at one site or distributed across multiple sites and interconnected by a communication network

A computer can be embedded in another device, *e.g.,* a mobile telephone, a personal digital assistant (PDA), a mobile audio player, a Global Positioning System (GPS) receiver, to name just a few. Computer-readable media suitable for storing computer program instructions and data include all forms of non-volatile memory, media and memory devices, including by way of example semiconductor memory devices, *e.g.*, EPROM, EEPROM, and flash memory devices; magnetic disks, *e.g*., internal hard disks or removable disks; magneto-optical disks; and CD-ROM and DVD-ROM disks.

To provide for interaction with a user, certain embodiments can be implemented on a computer having a display device, *e.g.,* a CRT (cathode ray tube) or LCD (liquid crystal display) monitor, for displaying information to the user and a keyboard and a pointing device, *e.g.,* a mouse or a trackball, by which the user can provide input to the computer. Other kinds of devices can be used to provide for interaction with a user as well; for example, feedback provided to the user can be any form of sensory feedback, *e.g*., visual feedback, auditory feedback, or tactile feedback; and input from the user can be received in any form, including acoustic, speech, or tactile input.

In certain embodiments, the display and/or user interface can be configured to display one or more recommendations generated according to the methods of the invention. The display or user interface can also be configured to display purchasing instructions based on the recommendations and/or a ranking of the pre-manufactured food products, allowing a user or other external device to submit purchasing requests for the food products. The display or user interface can also be configured to display shipping information for one or more purchase requests for the food products.

Certain embodiments can be implemented in a computing system that includes a back-end component, *e.g.,* as a data server, or that includes a middleware component, *e.g.,* an application server, or that includes a front-end component, *e.g*., a client computer having a graphical user interface or a Web browser through which a user can interact with an certain embodiments, or any combination of one or more such back-end, middleware, or front-end components. The components of the system can be interconnected by any form or medium of digital data communication, *e.g*., a communication network. Examples of communication networks include a local area network ("LAN") and a wide area network ("WAN"), *e.g.,* the Internet.

Certain embodiments can be used for purchasing of the recommended food product or other products included in the ranking. The purchasing can be done automatically be the system, or otherwise initiated by an external source, such as an external computer or a user. In some embodiments, the system can submit a purchase requests for the recommended food product or any of the ranked food products. Further, the methods according to certain embodiments can include displaying shipping information for the one or more purchase requests.

In certain embodiments, an algorithm can be used for one or more of providing a nutritional profile 140, ranking the selected pre-manufactured food product(s) 185, and/or providing a recommendation from the selected pre-manufactured food products 190. Food products 185/190 can be any pet food or animal food described herein or known in the art. For example, the algorithm can first generate a plurality of nutritional criteria. Each of the plurality of nutritional criteria can be assigned a criteria weight value. Each of the criteria can then compared against ingredients or other factors stored within a first database 220 and/or a prioritization value can be assigned to each of the plurality of criteria. Criteria falling above a certain prioritization value are then used to calculate the nutritional profile. Each criteria further comprises a min, max, target, and weight value for a plurality of potential ingredients.

After the nutritional criteria is generated using first database 220, an algorithm can rank selected pre-manufactured food products from a second database 230 and/or provide a recommendations from the selected pre-manufactured food products. The second database can include a plurality of pre-manufactured food products, each of the plurality of pre-manufactured food products further comprising a plurality of ingredients. The algorithm can generate a list of each of the plurality of ingredients and compares them with each of the min, max, target, and weight value for the plurality of potential ingredients for each criteria. A nutritional score can be generated for each product, based on the comparison of each ingredient of the product compared to the min, max, target, and weight value for each of the plurality of ingredients. The products can then be ranked based on the plurality of criteria as compared to the plurality of ingredients in each product. In certain embodiments, the algorithm can produce a recommendation based on the product rankings.

Certain embodiments provide systems, methods, media and combinations thereof for using machine learning systems or models to generate a nutritional profile, ranking selected pre-manufactured food products, and/or determining a recommendation from the selected pre-manufactured food products. The algorithm discussed above, for example, can take the form of any of the machine learning models describes herein. In some embodiments, the machine learning model or tool can be a K-nearest neighbor (KNN), naive Bayes (NB), decision trees or random forests, support vector machine (SVM), a deep learning model, such as convolutional neural network (CNN), region-based CNN (RCNN), one-dimensional (1-D) CNN, recurrent neural networks (RNNs), or any other machine learning model or technique. The machine learning system or tool can be trained to determine nutritional profiles 140, rank food products 185, and/or recommend food products. In certain embodiments, a list of ingredients and corresponding nutritional criteria can be used to train a machine learning system or model, such that the system or model can determine correlations between ingredients and corresponding nutritional criteria. The system or model can be trained to compare a list of ingredients to a set of nutritional criteria to determine food products which meet a pre-determined set of nutritional criteria. In other embodiments, the system or model can be trained to recommend one or more food products based on the nutritional profile of an animal or pet.

The invention is restricted to the subject-matter of the claims.

### EXAMPLES

### Example 1

### Nutritional profile for a pathologic adult neutered dog

This example takes into consideration an adult dog for which an individual physiological profile and an individual pathological profile were provided as follows:

**Table 2 - Individual profile**

| **Species** | **Dog** |
|---|---|
| Breed | Mix breed medium size |
| Neutering Status | Neutered |
| Birth Date | 2015 (Adult lifestage) |
| BCS (Body condition score) | 8 |
| Weight | 18 kg |
| Pathologies/Diseases | Obesity (BCS 8 out of 9) & Osteochondritis |

Based on the individual profile of the dog, a nutritional profile (or nutritional recommendation) is provided hereafter for each general criteria, such as lifestage, risk factor and/or disease.

**Table 3 - Ingredient recommendation vs. lifestage (adulthood)**

| | | **LIFESTAGE** | | | **Weight** |
|---|---|---|---|---|---|
| | | ADULTHOOD | | | 2 |
| **Ingredient name** | **unit** | **MIN** | **TARGET** | **MAX** | |
| Ca/P | ratio | 1 | 1,3 | 2 | 3 |
| PROT | g/Mcal | 50 | 65 | | 3 |
| FAT | g/Mcal | 30 | 36 | 82,5 | 3 |
| Ca | g/Mcal | 1,8 | 2,5 | 7,5 | 2 |
| P | g/Mcal | 1,2 | 2 | | 2 |
| Na | g/Mcal | 0,7 | 1 | 3,75 | 2 |
| Cl | g/Mcal | 0,9 | 2,5 | 5,625 | 2 |
| K | g/Mcal | 1,5 | 1,8 | | 2 |
| Mg | g/Mcal | 0,12 | 0,23 | | 2 |
| Fe | mg/Mcal | 20 | 35 | 355 | 2 |
| Cu | mg/Mcal | 2,5 | 4 | 37,5 | 2 |
| Zn | mg/Mcal | 35 | 50 | 250 | 2 |
| Mn | mg/Mcal | 10 | 17 | 200 | 2 |
| I | mg/Mcal | 0,7 | 1,1 | 12,5 | 2 |
| Se | mg/Mcal | | 0,12 | 0,25 | 2 |
| LINOLEIC | g/Mcal | 5 | 8 | | 2 |
| ARG | g/Mcal | 2,2 | 4 | | 2 |
| HIS | g/Mcal | 1 | 1,3 | | 2 |
| ILE | g/Mcal | 1,8 | 2,6 | | 2 |
| LEU | g/Mcal | 3,5 | 6 | | 2 |
| MET+CYS | g/Mcal | 2,23 | 2,5 | | 2 |
| MET | g/Mcal | 1,2 | 1,5 | | 2 |
| PHE | g/Mcal | 2 | 3 | | 2 |
| PHE+TYR | g/Mcal | 4 | 5,4 | | 2 |
| THR | g/Mcal | 1,59 | 2,5 | | 2 |
| TRP | g/Mcal | 0,48 | 0,58 | | 2 |
| VAL | g/Mcal | 2 | 3,1 | | 2 |
| CHOLINE | mg/Mcal | 550 | 600 | | 2 |
| LYS | g/Mcal | 2 | 2,7 | | 2 |
| VIT A | UI/Mcal | 3500 | | | 2 |
| VIT D3 | UI/Mcal | 200 | 250 | | 2 |
| VIT E | mg/Mcal | 100 | 130 | | 2 |
| B1 | mg/Mcal | 1 | 1,3 | | 2 |
| B2 | mg/Mcal | 1,35 | 1,65 | | 2 |
| B5 | mg/Mcal | 5,5 | 7,9 | | 2 |
| B3 | mg/Mcal | 7,5 | 11 | | 2 |
| B6 | mg/Mcal | 1,7 | 2,6 | | 2 |
| B9 | mg/Mcal | 0,15 | 0,2 | | 2 |
| B7 | mg/Mcal | 0,1 | 0,28 | | 2 |
| B12 | mg/Mcal | 0,012 | 0,03 | | 2 |

**Table 4 - Ingredient recommendation vs. Risk factor**

| | | **RISK FACTOR** | | | **Weight** |
|---|---|---|---|---|---|
| | | ANTIOXIDANT (AOX) IMMUNO ADULT | | | 1 |
| **ingredient name** | **unit** | **MIN** | **TARGET** | **MAX** | |
| TAU | g/Mcal | 0,5 | | | 2 |
| VIT E | mg/Mcal | 120 | 130 | | 2 |
| VIT E | mg/Mcal | | | | |
| VIT C | mg/Mcal | 42 | 50 | | 2 |
| LUTEIN | mg/Mcal | 0,7 | | | 2 |

**Table 5 - Ingredient Recommendation vs. diseases**

| | | **DISEASES** | | | | **DISEASES** | | | |
|---|---|---|---|---|---|---|---|---|---|
| | | OSTEOARTHRITIS + OTHER MOBILITY | | | **Weight** | OVERWEIGHT & OBESE (ACTIVE | | | **Weight** |
| | | CONDITIONS | | | **4** | WEIGHT LOSS) | | | **4** |
| **ingredient name** | **unit** | **MIN** | **TARGET** | **MAX** | | **MIN** | **TARGET** | **MAX** | |
| Ca / P | ratio | | | | | 1 | 1,3 | 2 | 2 |
| PROT | g/Mcal | | | | | 96 | 108 | 120 | 3 |
| FAT | g/Mcal | | | | | 28 | 34 | 41,3 | 2 |
| Ca | g/Mcal | | | | | 2,3 | 3,3 | 4,5 | 2 |
| P | g/Mcal | | | | | 1,39 | 2,5 | 3,25 | 2 |
| Na | g/Mcal | | | | | 0,8 | 1 | | 2 |
| Cl | g/Mcal | | | | | 2 | 3 | | 2 |
| K | g/Mcal | | | | | 2,35 | 2,8 | | 2 |
| Mg | g/Mcal | | | | | 0,18 | 0,4 | | 2 |
| Fe | mg/Mcal | | | | | 39 | 50 | 125 | 2 |
| Cu | mg/Mcal | | | | | 4 | 5,9 | | 2 |
| Zn | mg/Mcal | | | | | 40 | 70 | | 2 |
| Mn | mg/Mcal | | | | | 18 | 25 | | 2 |
| I | mg/Mcal | | | | | 1,3 | 1,8 | | 2 |
| Se | mg/Mcal | | | | | 0,1 | 0,15 | | 2 |
| LINO+ARACH | g/Mcal | | | | | 5,5 | 7 | | 2 |
| LINOLEIC | g/Mcal | | | | | 4 | 6,8 | | 2 |
| ARACH | g/Mcal | | | | | 0,1 | 0,25 | | 1 |
| ALA | g/Mcal | | | | | | 0,65 | | 1 |
| ARG | g/Mcal | | | | | 4,7 | 5,6 | | 2 |
| HIS | g/Mcal | | | | | 1,85 | 2,2 | | 2 |
| ILE | g/Mcal | | | | | 3,4 | 3,9 | | 2 |
| LEU | g/Mcal | | | | | 7 | 9 | | 2 |
| MET+CYS | g/Mcal | | | | | 3,5 | 4,2 | | 2 |
| MET | g/Mcal | | | | | 2 | 3 | | 2 |
| PHE | g/Mcal | | | | | 4,1 | 4,9 | | 2 |
| PHE+TYR | g/Mcal | | | | | 7,2 | 8,5 | | 2 |
| THR | g/Mcal | | | | | 3 | 3,7 | | 2 |
| TRP | g/Mcal | | | | | 0,75 | 0,95 | | 2 |
| VAL | g/Mcal | | | | | 3,9 | 4,7 | | 2 |
| CHOLINE | mg/Mcal | | | | | 615 | 1200 | | 3 |
| TDF | g/Mcal | | | | | 54 | 99 | | 3 |
| POLYPH GTEA | mg/Mcal | 200 | 220 | | 3 | | | | |
| EPA+DHA | g/Mcal | 1,5 | 1,8 | | 3 | 0,8 | 1,2 | | 2 |
| LYS | g/Mcal | | | | | 3 | 4,2 | | 2 |
| VIT A | UI/Mcal | | | | | 6100 | 7750 | | 2 |
| VIT D3 | UI/Mcal | | | | | 290 | 365 | | 2 |
| VIT E | mg/Mcal | | | | | 127 | 270 | | 2 |
| B1 | mg/Mcal | | | | | | 6,25 | | 2 |
| B2 | mg/Mcal | | | | | | 23,5 | | 2 |
| B5 | mg/Mcal | | | | | | 22 | | 2 |
| B3 | mg/Mcal | | | | | | 80 | | 2 |
| B6 | mg/Mcal | | | | | | 10,3 | | 2 |
| B9 | mg/Mcal | | | | | | 1,67 | | 2 |
| B7 | mg/Mcal | | | | | | 0,67 | | 2 |
| B12 | mg/Mcal | | | | | | 0,065 | | 2 |
| CURCUMINOIDS | mg/Mcal | 75 | 88 | | | | | | |
| Collagene hydro | g/Mcal | 3,5 | 4,3 | | | | | | |
| LCARNIT | ppm | | | | | | 320 | | 3 |

Only the ingredient names which were assigned a value range were defined in the above-mentioned tables. In this case, the neutered weight values are depriorized due to the presence of an overweight & obese condition associated to the same ingredients.

### Example 2

### Nutritional profile for a pathologic adult non neutered cat

This example takes into consideration an adult cat for which an individual physiological profile and an individual pathological profile were provided as follows:

**Table 6 - Individual cat profile**

| | |
|---|---|
| Species | Cat |
| Breed | European |
| Neutering Status | Not Neutered |
| Birth Date | 2015 (Adult lifestage) |
| BCS (Body condition score) | 5 |
| Weight | 18 kg |
| Pathologies/Diseases | Congestive heart failure & Azotemic chronic kidney disease (IRIS - International Renal Interest Society stage 3) |

**Table 7 - Ingredient Recommendation vs. lifestage**

| | | **LIFESTAGE** | | | **Weight** |
|---|---|---|---|---|---|
| | | ADULTHOOD | | | 1 |
| **ingredient name** | **unit** | **MIN** | **TARGET** | **MAX** | |
| P | g/Mcal | 0,77 | 2,3 | | 3 |
| PROT | g/Mcal | 60,9 | 85 | | 3 |
| FAT | g/Mcal | 27,4 | 40 | 82,5 | 3 |
| Ca | g/Mcal | 0,87 | 2,67 | | 2 |
| Ca/P | ratio | 1 | 1,15 | 2 | 2 |
| Na | g/Mcal | 0,21 | 1,5 | 4,5 | 2 |
| Cl | g/Mcal | 0,29 | 2,4 | 6,5 | 2 |
| K | g/Mcal | 1,7 | 1,85 | | 2 |
| Mg | g/Mcal | 0,1 | 0,2 | 0,43 | 2 |
| Fe | mg/Mcal | 24 | 35 | 125 | 2 |
| Cu | mg/Mcal | 1,5 | 5 | | 2 |
| Zn | mg/Mcal | 22,5 | 45 | 150 | 2 |
| Mn | mg/Mcal | 1,5 | 15 | | 2 |
| I | mg/Mcal | 0,4 | 1,1 | 5,28 | 2 |
| Se | mg/Mcal | 0,06 | 0,09 | | 2 |
| LINOLEIC | g/Mcal | 1,7 | 9,5 | 13,8 | 2 |
| ARACH | g/Mcal | 0,02 | 0,2 | 0,5 | 2 |
| ARG | g/Mcal | 2,15 | 4,1 | | 2 |
| HIS | g/Mcal | 0,79 | 1,6 | | 2 |
| ILE | g/Mcal | 1,31 | 2,9 | | 2 |
| LEU | g/Mcal | 3,1 | 6,1 | | 2 |
| MET+CYS | g/Mcal | 1,03 | 3,7 | | 2 |
| MET | g/Mcal | 0,52 | 2,4 | | 2 |
| PHE | g/Mcal | 1,22 | 3,3 | | 2 |
| PHE+TYR | g/Mcal | 4,66 | 6 | | 2 |
| THR | g/Mcal | 1,6 | 2,5 | | 2 |
| TRP | g/Mcal | 0,4 | 0,7 | | 2 |
| VAL | g/Mcal | 1,56 | 3,5 | | 2 |
| CHOLINE | mg/Mcal | 536 | 755 | | 2 |
| EPA+DHA | g/Mcal | | 0,75 | | 1 |
| LYS | g/Mcal | 1,03 | 3,5 | | 2 |
| TAU | g/Mcal | 0,37 | 0,5 | | 3 |
| VIT A | UI/Mcal | 1014 | 6000 | 83325 | 2 |
| VIT D3 | UI/Mcal | 85 | 200 | 7520 | 2 |
| VIT E | mg/Mcal | 12,2 | 161 | | 2 |
| B1 | mg/Mcal | 1,35 | 6 | | 2 |
| B2 | mg/Mcal | 1,22 | 13 | | 2 |
| B5 | mg/Mcal | 2,59 | 35 | | 2 |
| B3 | mg/Mcal | 12,2 | 125 | | 2 |
| B6 | mg/Mcal | 0,76 | 18 | | 2 |
| B9 | mg/Mcal | 0,23 | 3,2 | | 2 |
| B7 | mg/Mcal | 0,02 | 0,79 | | 2 |
| B12 | mg/Mcal | 0,01 | 0,035 | | 2 |

**Table 8 - Ingredient Recommendation vs. risk factors**

| | | **RISK FACTOR** | | | **Weight** |
|---|---|---|---|---|---|
| | | AOX IMMUNO ADULT | | | 1 |
| **ingredient name** | **unit** | **MIN** | **TARGET** | **MAX** | |
| TAU | g/Mcal | | 0,65 | | 2 |
| VIT E | mg/Mcal | | 130 | | 2 |
| VIT C | mg/Mcal | | 55 | | 2 |
| LUTEIN | mg/Mcal | | 1,3 | | 3 |

**Table 9 - Ingredient Recommendation vs. disease**

| | | **DISEASES** | | | **Weight** | **DISEASES** | | | **Weight** |
|---|---|---|---|---|---|---|---|---|---|
| | | CARDIAC - CONGESTIVE HEART FAILURE | | | 4 | IRIS STAGE III | | | 5 |
| **ingredient name** | **unit** | **MIN** | **TARGET** | **MAX** | | **MIN** | **TARGET** | **MAX** | |
| P | g/Mcal | | | | | 0,79 | 0,9 | 1,2 | O |
| PROT | g/Mcal | | | | | 55 | 58 | 65 | O |
| Ca | g/Mcal | | | | | 1,1 | 1,5 | | 2 |
| Na | g/Mcal | | 0,5 | 0,7 | 3 | 0,5 | 0,9 | 1,1 | O |
| Cl | g/Mcal | | 1 | 3 | 3 | | | | |
| K | g/Mcal | | | | | 1,8 | 2,3 | | 2 |
| EPA+DHA | g/Mcal | 0,9 | 1 | | 2 | | | | |

### Example 3

### Illustration of the calculation for the ranking in the first database

This example can comprise a hypothetical example starting from 3 nutritional criteria. It has no objective other than just to illustrate a calculation mode which can be used according to the present application.

### A - Pet description:

**Table 10 - Pet description**

| **Field description** | **Field Value** | **Nutritional Criteria** |
|---|---|---|
| Species | Cat | |
| Breed | European | |
| Neutering Status | Neutered | Criteria 2 |
| Lifestage | Adult | Criteria 1 |
| Pathologies/Diseases | Congestive heart failure | Criteria 3 |

### B - First database used for the generation of the nutritional profile contains the following tables:

**Table 11 - Nutritional criteria**

| | **Criteria 1 - (weight 5)** | | | | **Criteria 2 - (weight 2)** | | | | **Criteria 3 - (weight 4)** | | | |
|---|---|---|---|---|---|---|---|---|---|---|---|---|
| | **Min** | **Max** | **Target** | **Weight** | **Min** | **Max** | **Target** | **Weight** | **Min** | **Max** | **Target** | **Weight** |
| Ingredient 1 | 95 | | 100 | 3 | | | | | | | | |
| Ingredient 2 | 1 | | max | 2 | | | | | 0.8 | | 1.2 | 2 |
| Ingredient 3 | | | | | 72 | | 86 | 3 | 96 | 120 | 108 | 3 |
| Ingredient 4 | | | | | | | | | 2.3 | 4.5 | 3.3 | 2 |

According to the description of the pet described in A the 3 nutritional criteria above are called by the algorithm.

**Table 12 - Prioritization**

| **PRIORITIZATION** | **CRITERIA** |
|---|---|
| 1 | Criteria 2 |
| 2 | Criteria 3 |

In this case, *"Criteria 3"* has priority over *"Criteria 2".* Only *"Criteria 3"* and *"Criteria 1"* are used to calculate the **nutritional profile.**
C - **Second database,** made of the product and ingredients, contains the table

**Table 13**

| | **Product 1** | **Product 2** | **Product 3** |
|---|---|---|---|
| Ingredient 1 | 100 | 99 | 0 |
| Ingredient 2 | 1.69 | 1.49 | 1.28 |
| Ingredient 3 | 108.84 | 77.35 | 109.15 |
| Ingredient 4 | 2.78 | 3.03 | 3.28 |

D - The optimization model calculates the nutritional profile as in table hereafter:

**Table 14**

| | **Min** | **Max** | **Target** | **Weight** |
|---|---|---|---|---|
| Ingredient 1 | 95 | 101 | 100 | 100000000 |
| Ingredient 2 | 1 | 2.4 | 2.35 | 10100000 |
| Ingredient 3 | 96 | 120 | 108 | 1000000 |
| Ingredient 4 | 2 | 4.5 | 3.3 | 100000 |

Min, Max, Target and Weight values from Criteria 1 and Criteria 3 are combined together to generate this profile with our optimization algorithm.
E - Products ranking and weighted percentage of coverages:
After projection of the nutritional profile from D on the product database from C the products are ranked as follow:

**Table 15 - Products ranking**

| **Coverage** | **Product 1** | **Product 2** | **Product 3** |
|---|---|---|---|
| Overall | 100 | 99.52 | 17.5 |
| Criteria 1 | 100 | 100 | 16.7 |
| Criteria 2 | 100 | 97.6 | 100 |
| Criteria 3 | 100 | 51.8 | 100 |

Products columns are ranked in the order of best matched products for the nutritional profile. In certain embodiments the ranking can be performed using a machine learning model or tool.

## Claims

1. A computer-implemented method for characterizing the nutritional needs of an animal comprising:
providing an animal profile comprising one or more animal criteria;
extracting, from a first database, one or more lists of ingredients associated with the one or more animal criteria, wherein each one of the ingredients is assigned a range amount, wherein the range comprises a minimum amount and maximum amount, a minimum amount and a target amount, a maximum amount and a target amount, or a minimum amount, a maximum amount and a target amount;
determining a rating associated with the extracted lists of ingredients, wherein the rating provides the importance of the ingredients compared to the importance of other ingredients for the animal profile;
comparing the rating with at least one ingredient from the one or more lists of ingredients;
determining a nutritional profile of the animal based on the animal profile, the extracted lists of ingredients, and the rating of the extracted lists of ingredients.

2. The computer-implemented method of claim 1, further comprising:
extracting, from a second database, a list of food products;
selecting at least one food product from the list of food products based on the nutritional profile;
ranking the selected food products;
determining a recommended food product based on the ranking of the selected food products.

3. The computer-implemented method according to claim 2, wherein the food products are pre-manufactured food products.

4. The computer-implemented method of any of claims 2-3, wherein the food products are selected from a group comprising: dry food products, wet food products, semi-moist food products, treats, and Functional Meal Enhancers.

5. The computer-implemented method of any of claims 2-4, wherein the ranking further comprises:
assigning to one or more of the selected food products a quality factor based on the nutritional profile;
ranking the selected food products based on the quality factor, by reference to at least one non-selected food product of the second database.

6. The computer-implemented method of any of claims 2-5, further comprising:
displaying the recommended food product on a user display.

7. The computer-implemented method of any of claims 1-6, wherein the one or more animal criteria comprises at least one of a species, an activity level, a medical history, a breed, a reproductive status, a feeding method, an age, a sex, a spayed or neutered status, a meal schedule, a biological value from a biological sample, a body condition, a health status, a life style, a coat information, a risk factor, a weight of the animal.

8. The computer-implemented method of any of claims 1-7, wherein determining the nutritional profile further comprises:
generating one or more of nutritional criteria;
assigning a criteria value to of the one or more nutritional criteria;
comparing the criteria value against the list of ingredients;
calculating the nutritional profile based on at least one of the nutritional criteria, the list of ingredients, and the criteria value.

9. The computer-implemented method of any of claims 1-8, wherein determining the nutritional profile further comprises:
assigning a prioritization value to the one or more nutritional criteria;
calculating the nutritional value based on at least one of the nutritional criteria, the list of ingredient, the criteria value, and the prioritization value.

10. A system comprising means for characterizing the nutritional needs of an animal according to claim 1, the system (200) comprising:
a processor (202);
a storage unit (206);
a memory (204);
a user interface (210) illustrated on a display; and
a first database (220), comprising one or more lists of ingredients associated with one or more animal criteria,
wherein the memory comprises instructions, which when executed by the processor, are configured to cause the system to:
provide an animal profile comprising one or more animal criteria;
extract, from the first database, the one or more lists of ingredients, wherein each one of the ingredients is assigned a range amount, defined by at least one of a minimum amount, a maximum amount, or a target amount;
determine a rating associated with the extracted lists of ingredients;
comparing the rating with at least one ingredient from the one or more lists of ingredients;
determine a nutritional profile of the animal, based on the animal profile and the one or more lists of ingredients.

11. The system of claim 10, further comprising:
a second database (230) comprising a plurality of food products, wherein the instructions are further configured to cause the system to:
extract, from the second database, a list of food products; select at least one food product from the second database based on the nutritional profile;
rank the selected food products;
determine a recommended food product based on the rank of each selected food product.

12. The system of claim 11, the user interface is further configured to display the recommended food product.

13. The system of any of claims 11-12, wherein the user interface is further configured to display a purchase screen based on the recommended food product.

14. The system of any of claim 11-13, wherein the instructions are further configured to place a purchase order for the recommended food product.

15. The system of any of claims 11-14, wherein the food products are pre-manufactured food products.

16. The system of any of claims 11-15, wherein the food products are at least one of dry food products, wet food products, semi-moist food products, treats, and Functional Meal Enhancers.

17. The system of any of claims 10-16, wherein the one or more animal criteria are selected from at least one of a species, an activity level, a medical history, a breed, a reproductive status, a feeding method, an age, a sex, a spayed or neutered status, a meal schedule, a biological value from a biological sample, a body condition, a health status, a life style, a coat information, a risk factor, a weight of the animal.

## Patentansprüche

1. Computerimplementiertes Verfahren zum Charakterisieren des Nährstoffbedarfs eines Tieres, das Folgendes umfasst:
Bereitstellen eines Tierprofils, das ein oder mehrere Tierkriterien umfasst;
Extrahieren, aus einer ersten Datenbank, einer oder mehrerer Zutatenlisten, die mit dem einen oder den mehreren Tierkriterien assoziiert sind, wobei jeder der Zutaten ein Mengenbereich zugewiesen wird, wobei der Bereich eine Mindestmenge und eine Höchstmenge, eine Mindestmenge und eine Zielmenge, eine Höchstmenge und eine Zielmenge oder eine Mindestmenge, eine Höchstmenge und eine Zielmenge umfasst;
Bestimmen einer mit den extrahierten Zutatenlisten assoziierten Bewertung, wobei die Bewertung die Wichtigkeit der Zutaten im Vergleich zur Wichtigkeit anderer Zutaten für das Tierprofil angibt;
Vergleichen der Bewertung mit mindestens einer Zutat aus der einen oder den mehreren Zutatenlisten;
Bestimmen eines Nährstoffprofils des Tieres basierend auf dem Tierprofil, den extrahierten Zutatenlisten und der Bewertung der extrahierten Zutatenlisten.

2. Computerimplementiertes Verfahren nach Anspruch 1, das ferner Folgendes umfasst:
Extrahieren, aus einer zweiten Datenbank, einer Futterproduktliste;
Auswählen mindestens eines Futterprodukts aus der Futterproduktliste basierend auf dem Nährstoffprofil;
Einstufen der ausgewählten Futterprodukte;
Bestimmen eines empfohlenen Futterprodukts basierend auf der Einstufung der ausgewählten Futterprodukte.

3. Computerimplementiertes Verfahren nach Anspruch 2, wobei die Futterprodukte vorgefertigte Futterprodukte sind.

4. Computerimplementiertes Verfahren nach einem der Ansprüche 2-3, wobei die Futterprodukte aus einer Gruppe ausgewählt sind, die Folgendes umfasst:
Trockenfutterprodukte, Nassfutterprodukte, halbfeuchte Futterprodukte, Leckerlis und funktionelle Mahlzeitenverbesserer.

5. Computerimplementiertes Verfahren nach einem der Ansprüche 2-4, wobei die Einstufung ferner Folgendes umfasst:
Zuweisen eines Qualitätsfaktors zu einem oder mehreren der ausgewählten Futterprodukte basierend auf dem Nährstoffprofil;
Einstufen der ausgewählten Futterprodukte basierend auf dem Qualitätsfaktor unter Bezugnahme auf mindestens ein nicht ausgewähltes Futterprodukt aus der zweiten Datenbank.

6. Computerimplementiertes Verfahren nach einem der Ansprüche 2-5, das ferner Folgendes umfasst:
Anzeigen des empfohlenen Futterprodukts auf einer Benutzeranzeige.

7. Computerimplementiertes Verfahren nach einem der Ansprüche 1-6, wobei das eine oder die mehreren Tierkriterien mindestens eines einer Tierart, eines Aktivitätsniveaus, einer Krankengeschichte, einer Rasse, eines Fortpflanzungsstatus, eines Fütterungsverfahrens, eines Alters, eines Geschlechts, eines Sterilisations- oder Kastrationsstatus, eines Mahlzeitenplans, eines biologischen Werts aus einer biologischen Probe, einer körperlichen Verfassung, eines Gesundheitsstatus, eines Lebensstils, einer Fellinformation, eines Risikofaktors, eines Gewichts des Tieres umfasst.

8. Computerimplementiertes Verfahren nach einem der Ansprüche 1-7, wobei das Bestimmen des Nährstoffprofils ferner Folgendes umfasst:
Erzeugen eines oder mehrerer Nährstoffkriterien;
Zuweisen eines Kriterienwerts zu dem einen oder den mehreren Nährstoffkriterien;
Vergleichen des Kriterienwerts mit der Zutatenliste;
Berechnen des Nährstoffprofils basierend auf mindestens einem der Nährstoffkriterien, der Zutatenliste und des Kriterienwerts.

9. Computerimplementiertes Verfahren nach einem der Ansprüche 1-8, wobei das Bestimmen des Nährstoffprofils ferner Folgendes umfasst:
Zuweisen eines Priorisierungswerts zu dem einen oder den mehreren Nährstoffkriterien;
Berechnen des Nährstoffwerts basierend auf mindestens einem der Nährstoffkriterien, der Zutatenliste, des Kriterienwerts und des Priorisierungswerts.

10. System, das Mittel zum Charakterisieren des Nährstoffbedarfs eines Tieres nach Anspruch 1 umfasst, wobei das System (200) Folgendes umfasst:
einen Prozessor (202);
eine Speichereinheit (206);
einen Speicher (204);
eine Benutzerschnittstelle (210), die auf einer Anzeige dargestellt wird; und
eine erste Datenbank (220), die eine oder mehrere Zutatenlisten umfasst, die mit einem oder mehreren Tierkriterien assoziiert sind,
wobei der Speicher Anweisungen umfasst, die, wenn sie von dem Prozessor ausgeführt werden, dazu konfiguriert sind,
das System zu Folgendem zu veranlassen:
Bereitstellen eines Tierprofils, das ein oder mehrere Tierkriterien umfasst;
Extrahieren, aus der ersten Datenbank, der einen oder der mehreren Zutatenlisten, wobei jeder der Zutaten ein Mengenbereich zugewiesen ist, der durch mindestens eines einer Mindestmenge, einer Höchstmenge oder einer Zielmenge definiert ist;
Bestimmen einer Bewertung, die mit den extrahierten Zutatenlisten assoziiert ist;
Vergleichen der Bewertung mit mindestens einer Zutat aus der einen oder den mehreren Zutatenlisten;
Bestimmen eines Nährstoffprofils des Tieres basierend auf dem Tierprofil und der einer oder den mehreren Zutatenlisten.

11. System nach Anspruch 10, das ferner Folgendes umfasst:
eine zweite Datenbank (230), die eine Mehrzahl von Futterprodukten umfasst, wobei die Anweisungen ferner dazu konfiguriert sind, das System zu Folgendem zu veranlassen:
Extrahieren, aus der zweiten Datenbank, einer Futterproduktliste;
Auswählen mindestens eines Futterprodukts aus der zweiten Datenbank basierend auf dem Nährstoffprofil;
Einstufen der ausgewählten Futterprodukte;
Bestimmen eines empfohlenen Futterprodukts basierend auf der Einstufung jedes ausgewählten Futterprodukts.

12. System nach Anspruch 11, wobei die Benutzerschnittstelle ferner dazu konfiguriert ist, das empfohlene Futterprodukt anzuzeigen.

13. System nach einem der Ansprüche 11-12, wobei die Benutzerschnittstelle ferner dazu konfiguriert ist, basierend auf dem empfohlenen Futterprodukt einen Kaufbildschirm anzuzeigen.

14. System nach einem der Ansprüche 11-13, wobei die Anweisungen ferner dazu konfiguriert sind, einen Kaufauftrag für das empfohlene Futterprodukt aufzugeben.

15. System nach einem der Ansprüche 11-14, wobei die Futterprodukte vorgefertigte Futterprodukte sind.

16. System nach einem der Ansprüche 11-15, wobei die Futterprodukte mindestens eines von Trockenfutterprodukten, Nassfutterprodukten, halbfeuchten Futterprodukten, Leckerlis und funktionellen Mahlzeitenverbesserern sind.

17. System nach einem der Ansprüche 10-16, wobei das eine oder die mehreren Tierkriterien aus mindestens einem einer Tierart, eines Aktivitätsniveaus, einer Krankengeschichte, einer Rasse, eines Fortpflanzungsstatus, eines Fütterungsverfahrens, eines Alters, eines Geschlechts, eines Sterilisations- oder Kastrationsstatus, eines Mahlzeitenplans, eines biologischen Werts aus einer biologischen Probe, einer körperlichen Verfassung, eines Gesundheitsstatus, eines Lebensstils, einer Fellinformation, eines Risikofaktors, eines Gewichts des Tieres ausgewählt sind.

## Revendications

1. Procédé mis en œuvre par ordinateur pour caractériser les besoins nutritionnels d'un animal comprenant :
la fourniture d'un profil d'animal comprenant un ou plusieurs critères d'animal ;
l'extraction, d'une première base de données, d'une ou de plusieurs listes d'ingrédients associés à l'un ou aux plusieurs critères d'animal, dans lequel à chacun des ingrédients est attribué une quantité de plage, dans lequel la plage comprend une quantité minimum et quantité maximum, une quantité minimum et une quantité cible, une quantité maximum et une quantité cible, ou une quantité minimum, une quantité maximum et une quantité cible ;
la détermination d'une notation associée aux listes extraites d'ingrédients, dans lequel la notation fournit l'importance des ingrédients par comparaison à l'importance d'autres ingrédients pour le profil d'animal ;
la comparaison de la notation à au moins un ingrédient de l'une ou des plusieurs listes d'ingrédients ;
la détermination d'un profil nutritionnel de l'animal sur la base du profil d'animal, des listes extraites d'ingrédients, et de la notation des listes extraites d'ingrédients.

2. Procédé mis en œuvre par ordinateur de la revendication 1, comprenant en outre :
l'extraction, d'une seconde base de données, d'une liste de produits alimentaires ;
la sélection d'au moins un produit alimentaire parmi la liste de produits alimentaires, sur la base du profil nutritionnel ;
le classement des produits alimentaires sélectionnés ;
la détermination d'un produit alimentaire recommandé, sur la base du classement des produits alimentaires sélectionnés.

3. Procédé mis en œuvre par ordinateur selon la revendication 2, dans lequel les produits alimentaires sont des produits alimentaires préfabriqués.

4. Procédé mis en œuvre par ordinateur de quelconques des revendications 2 et 3, dans lequel les produits alimentaires sont sélectionnés parmi un groupe comprenant : des produits alimentaires secs, des produits alimentaires humides, des produits alimentaires semi-humides, des récompenses, et des améliorateurs de repas fonctionnels.

5. Procédé mis en œuvre par ordinateur de quelconques des revendications 2 à 4, dans lequel le classement comprend en outre :
l'attribution, à un ou à plusieurs des produits alimentaires sélectionnés, d'un facteur de qualité sur la base du profil nutritionnel ;
le classement des produits alimentaires sélectionnés, sur la base du facteur de qualité, en référence à au moins un produit alimentaire non sélectionné de la seconde base de données.

6. Procédé mis en œuvre par ordinateur de quelconques des revendications 2 à 5, comprenant en outre :
l'affichage du produit alimentaire recommandé sur un dispositif d'affichage utilisateur.

7. Procédé mis en œuvre par ordinateur de quelconques des revendications 1 à 6, dans lequel l'un ou les plusieurs critères d'animal comprennent au moins un élément parmi une espèce, un niveau d'activité, des antécédents médicaux, une race, un état de reproduction, une méthode d'alimentation, un âge, un sexe, un état castré ou stérilisé, un programme de repas, une valeur biologique provenant d'un échantillon biologique, une condition corporelle, un état de santé, un mode de vie, des informations de robe, un facteur de risque, un poids de l'animal.

8. Procédé mis en œuvre par ordinateur de quelconques des revendications 1 à 7, dans lequel la détermination du profil nutritionnel comprend en outre :
la génération d'un ou de plusieurs critères nutritionnels ;
l'attribution d'une valeur de critères à l'un ou aux plusieurs critères nutritionnels ;
la comparaison de la valeur de critères à la liste d'ingrédients ;
le calcul du profil nutritionnel sur la base d'au moins un élément parmi les critères nutritionnels, la liste d'ingrédients, et la valeur de critères.

9. Procédé mis en œuvre par ordinateur de quelconques des revendications 1 à 8, dans lequel la détermination du profil nutritionnel comprend en outre :
l'attribution d'une valeur de mise en ordre de priorité à l'un ou aux plusieurs critères nutritionnels ;
le calcul de la valeur nutritionnelle sur la base d'au moins un élément parmi les critères nutritionnels, la liste d'ingrédient, la valeur de critères, et la valeur de mise en ordre de priorité.

10. Système comprenant des moyens pour caractériser les besoins nutritionnels d'un animal selon la revendication 1, le système (200) comprenant :
un processeur (202) ;
une unité de stockage (206) ;
une mémoire (204) ;
une interface utilisateur (210) illustrée sur un dispositif d'affichage ; et
une première base de données (220), comprenant une ou plusieurs listes d'ingrédients associés à un ou à plusieurs critères d'animal,
dans lequel la mémoire comprend des instructions, qui lorsqu'elles sont exécutées par le processeur, sont configurées pour amener le système :
à fournir un profil d'animal comprenant un ou plusieurs critères d'animal ;
à extraire, de la première base de données, l'une ou les plusieurs listes d'ingrédients, dans lequel à chacun des ingrédients est attribuée une quantité de plage, définie par au moins une quantité parmi une quantité minimum, une quantité maximum, ou une quantité cible ;
à déterminer une notation associée aux listes extraites d'ingrédients ;
à comparer la notation à au moins un ingrédient de l'une ou des plusieurs listes d'ingrédients ;
à déterminer un profil nutritionnel de l'animal, sur la base du profil d'animal et de l'une ou des plusieurs listes d'ingrédients.

11. Système de la revendication 10, comprenant en outre :
une seconde base de données (230) comprenant une pluralité de produits alimentaires, dans lequel les instructions sont en outre configurées pour amener le système :
à extraire, de la seconde base de données, une liste de produits alimentaires ;
à sélectionner au moins un produit alimentaire de la seconde base de données, sur la base du profil nutritionnel ;
à classer les produits alimentaires sélectionnés ;
à déterminer un produit alimentaire recommandé, sur la base du classement de chaque produit alimentaire sélectionné.

12. Système de la revendication 11, l'interface utilisateur étant en outre configurée pour afficher le produit alimentaire recommandé.

13. Système de quelconques des revendications 11 et 12, dans lequel l'interface utilisateur est en outre configurée pour afficher un écran d'achat, sur la base du produit alimentaire recommandé.

14. Système de quelconques des revendications 11 à 13, dans lequel les instructions sont en outre configurées pour faire une commande d'achat pour le produit alimentaire recommandé.

15. Système de quelconques des revendications 11 à 14, dans lequel les produits alimentaires sont des produits alimentaires préfabriqués.

16. Système de quelconques des revendications 11 à 15, dans lequel les produits alimentaires sont au moins un élément parmi des produits alimentaires secs, des produits alimentaires humides, des produits alimentaires semi-humides, des récompenses, et des améliorateurs de repas fonctionnels.

17. Système de quelconques des revendications 10 à 16, dans lequel l'un ou les plusieurs critères d'animal sont sélectionnés parmi au moins un élément parmi une espèce, un niveau d'activité, des antécédents médicaux, une race, un état de reproduction, une méthode d'alimentation, un âge, un sexe, un état castré ou stérilisé, un programme de repas, une valeur biologique provenant d'un échantillon biologique, une condition corporelle, un état de santé, un mode de vie, des informations de robe, un facteur de risque, un poids de l'animal.
